(19) Europäisches Patentamt
European Patent Office
Office européen des brevets

(11) Numéro de publication : **0 433 167 B1**

(12) # FASCICULE DE BREVET EUROPEEN

(45) Date de publication du fascicule du brevet : **02.03.94 Bulletin 94/09**

(51) Int. Cl.$^5$ : **C07D 211/54,** C07D 405/06, C07D 409/06, A61K 31/445

(21) Numéro de dépôt : **90403537.5**

(22) Date de dépôt : **12.12.90**

(54) **Nouveaux dérivés de la pipéridine, leur procédé de préparation et les compositions pharmaceutiques les renfermant.**

(30) Priorité : **12.12.89 FR 8916384**

(43) Date de publication de la demande : **19.06.91 Bulletin 91/25**

(45) Mention de la délivrance du brevet : **02.03.94 Bulletin 94/09**

(84) Etats contractants désignés : **AT BE CH DE DK ES FR GB GR IT LI LU NL SE**

(56) Documents cités :
**EP-A- 0 077 427**
**EP-A- 0 134 124**
**GB-A- 780 027**

(73) Titulaire : **ADIR ET COMPAGNIE**
**1 rue Carle Hébert**
**F-92415 Courbevoie Cédex (FR)**

(72) Inventeur : **Regnier, Gilbert**
**Demeure du Plessis, Bâtiment D, 27 av. du Plessis**
**F-92290 Chatenay Malabry (FR)**
Inventeur : **Renaud de la Faverie, J. F.**
**7 rue des Erables Rocquencourt**
**F-78150 Le Chesnay (FR)**
Inventeur : **Lombet, Alain**
**28 avenue René Damons**
**F-94500 Champigny (FR)**
Inventeur : **Iliou, Jean-Pierre**
**47 Boulevard Richard Wallace**
**F-92800 Puteaux (FR)**
Inventeur : **Vilaine, Jean-Paul**
**21 rue des Vallées**
**F-92290 Chatenay Malabry (FR)**
Inventeur : **Bidouard, Jean-Pierre**
**5 Résidence Croix Blanche**
**F-91380 Chilly Mazarin (FR)**
Inventeur : **Lenaers, Albert**
**20 Allée des Martinets**
**F-78510 Triel Sur Seine (FR)**

(74) Mandataire : **Reverbori, Marcelle**
**Adir et Compagnie, 1, rue Carle Hébert**
**F-92415 Courbevoie Cédex (FR)**

**EP 0 433 167 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet les nouveaux dérivés de la pipéridine, leur procédé de préparation et les compositions pharmaceutiques les renfermant.

Elle concerne particulièrement les dérivés de la pipéridine de formule générale I :

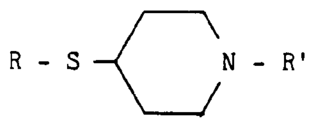

(I)

dans laquelle :
l'un des substituants R ou R' représente le radical de formule :

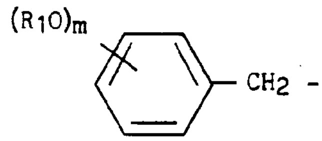

dans laquelle
m est un nombre entier de 1 à 3 et
$R_1$ représente un radical alkyle ayant de 1 à 5 atomes de carbone,
et
l'autre substituant R ou R' représente :
soit un radical de formule :

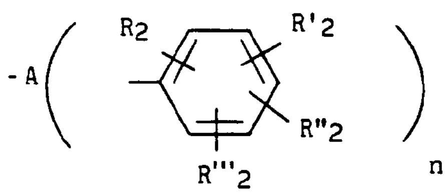

dans laquelle :

A représente une chaîne hydrocarbonée ayant de 1 à 5 atomes de carbone renfermant éventuellement une double liaison ou un atome d'oxygène ou de soufre, éventuellement substituée par un radical hydroxy ou un ou plusieurs radicaux méthyle ;
n prend les valeurs 1 ou 2 ;
et
$R_2$, $R'_2$, $R''_2$ et $R'''_2$ identiques ou différents, représentent chacun un atome d'hydrogène, un atome d'halogène tel qu'un atome de chlore ou de fluor, un radical alkyl ou alkoxy ayant chacun de 1 à 5 atomes de carbone en chaîne droite ou ramifiée, ou un radical hydroxy ;
soit un radical de formule :

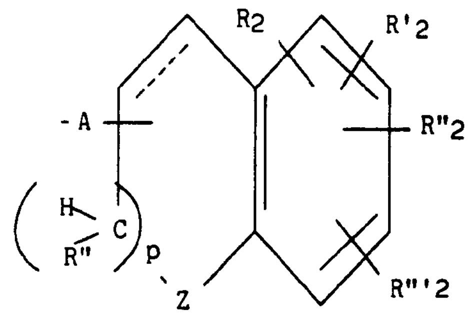

dans laquelle :

- Z représente un atome d'oxygène ou de soufre ;
- R" représente un atome d'hydrogène ou un radical méthyle ;
- p prend les valeurs zéro ou un
  et
- A, R2, $R'_2$, $R''_2$ et $R'''_2$ ont les significations précédemment définies.

L'état antérieur de la technique dans ce domaine est illustré notamment par la demande de brevet européen publiée sous le n° 0149088 qui concerne des dérivés de formule générale :

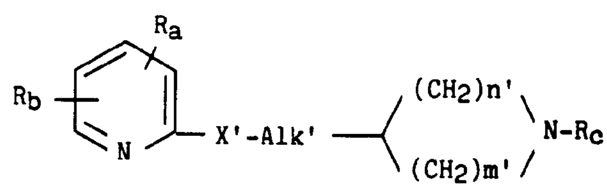

dans laquelle :

- $R_a$ et $R_b$ sont des substituants classiques
- X' est, entre autres, soufre
- Alk' est une chaîne alkylène de 0 à 4 atomes de carbone
- n' et m' identiques ou différents sont des nombres entiers de 1 à 3, et $R_c$ est, entre autres, hydrogène, ($C_1$-$C_6$) alkyle, ($C_3$-$C_6$) alkényle, ($C_3$-$C_6$) alkylnyle, ($C_3$-$C_7$) cycloalkyle, ($C_3$-$C_7$) cycloalkényle ;

lesquels dérivés ont des propriétés analgésiques.

Ce brevet européen ne décrit ni suggère les dérivés de la pipéridine objet de la présente invention, lesquels dérivés trouvent une application dans le traitement des pathologies liées à l'ischémie tissulaire, ce qui n'est pas le cas des dérivés de l'état antérieur de la technique ci-dessus cité.

La présente invention a également pour objet le procédé de préparation des dérivés de formule générale I, caractérisé en ce que l'on condense un dérivé de formule générale II :

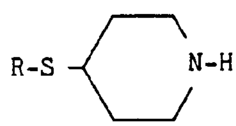

(II)

dans laquelle R a la signification précédemment définie, avec un dérivé de formule générale III :

Y-R' (III)

dans laquelle R' a la signification précédemment définie et Y représente un atome de chlore ou de brome, ou un radical tosyloxy.

La condensation s'effectue de façon particulièrement adéquate en opérant dans un solvant polaire tel que, par exemple, un alcool à bas poids moléculaire, le diméthylformamide ou le diméthylacétamide, ou dans un solvant aprotique, polaire tel que par exemple l'acétonitrile ou la méthyléthylcétone, en présence d'un accepteur de l'hydracide formé au cours de la réaction, à une température comprise entre 80 et 130°C.

Les matières premières de formule générale II peuvent être préparées selon la technique décrite dans le brevet européen n° 0149088, à partir du dérivé de formule IV :

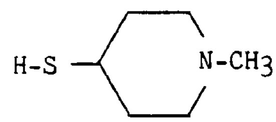

(IV)

lui-même préparé selon la méthode de H. BARRERA et R. LYLE J. Org. Chem 27, 641-643 (1962).

La méthode la plus adéquate consiste à condenser un dérivé de formule III sur le dérivé de formule IV préalablement sodé au moyen d'un hydrure alcalin dans un solvant aprotique polaire tel que le diméthylformamide ou le diméthylacétamide, ou au moyen d'un alcoolate alcalin dans un alcool à bas point d'ébullition, les solvants respectifs servant de milieu réactionnel, à des températures comprises entre 80 et 130°C.

Les dérivés obtenus de formule générale V :

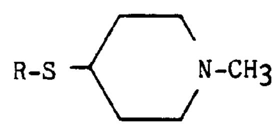

(V)

dans laquelle R a la signification précédemment définie, sont transformés en dérivés de formule générale VI :

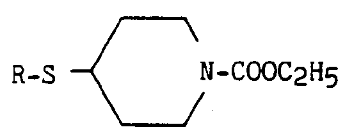

(VI)

dans laquelle R a la signification précédemment définie ; lesquels dérivés VI conduisent aux dérivés de formule générale II par hydrolyse au moyen d'HCl concentré ou de I $Si(CH_3)_3$ dans un solvant aprotique.

Les dérivés de formule générale I ont également été préparées selon une variante de la méthode précédemment décrite.

Ce procédé, qui est également inclus dans la présente invention, est caractérisé en ce que l'on condense un dérivé de formule générale IIa :

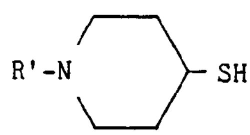

(IIa)

dans laquelle R' a la signification précédemment définie, avec un dérivé de formule générale IIIa:

Y-R (IIIa)

dans laquelle Y et R ont les significations précédemment définies.

La condensation s'effectue de façon particulièrement adéquate en opérant dans un solvant polaire tel qu'un alcool à bas point d'ébullition, le cyanure d'acétyle, la méthyléthylcétone, le diméthylformamide ou le diméthylacétamide, à une température comprise entre 80 et 110°C, en présence d'un agent alcalin tel que par exemple le carbonate de sodium ou de potassium.

La condensation peut également être réalisée à partir du dérivé IIa préalablement sodé au moyen d'un alcoolate ou d'un hydrure de sodium.

Les dérivés de formule générale I, obtenus selon les méthodes ci-dessus, peuvent être purifiés par cristallisation à l'état de sels ou de bases, ou par chromatoflash sur silice (35-70 µm) séparés par des systèmes tels que par exemple acétate d'éthyle-acétone, acétate d'éthyle-méthanol, acétate d'éthyle-dichlorométhane, dichlorométhane-méthanol ou dichlorométhane-cyclohexane.

Les dérivés de formule générale I donnent des sels avec les acides physiologiquement tolérables. Ces sels sont également inclus dans la présente invention.

Les dérivés de la présente invention possèdent des propriétés pharmacologiques et thérapeutiques intéressantes. En particulier, pour ces dérivés, ont été mises en évidence, in vitro : la protection cellulaire vis-à-vis des conséquences de l'acidose intra cellulaire et l'inhibition des canaux calciques lents ; et in vivo : la prévention et la limitation des effets induits par l'ischémie myocardiaque.

Ces propriétés permettent l'utilisation des dérivés de la présente invention comme médicament dans le traitement des pathologies liées à l'ischémie tissulaire, notamment dans le domaine cardiovasculaire : angine de poitrine, infarctus du myocarde, conséquences des cardiopathies ischémiques ; et de la pathologie vasculaire périphérique.

Les dérivés de la présente invention peuvent également être utilisés dans le domaine cérébral, notamment pour le traitement de l'accident vasculaire cérébral et des manifestations déficitaires liées aux troubles circulatoires cérébraux chroniques ; dans le domaine ophtalmologique : notamment pour le traitement des troubles rétiniens d'origine vasculaire ; et dans les manifestations neuro-sensorielles d'origine ischémique.

De plus, pour certains dérivés a été mise en évidence leur capacité à protéger, in vitro, d'une part les LDL humaines (lipoprotéines de faible densité assurant le transport du cholestérol) vis à vis des modifications oxydatives impliquées dans l'athérogénèse, et d'autre part les cellules cardiaques vis à vis d'une nécrose oxydative.

Ces propriétés rendent ces produits particulièrement intéressants pour la prévention ou le traitement des pathologies dans lesquelles une peroxydation lipidique joue un rôle initiateur et/ou aggravant : telles que, par exemple, les lésions vasculaires athérosclèreuses notamment dans le cadre des dyslipidémies, les cardiopathies ischémiques, la reperfusion d'organes y compris transplantés, les pathologies ischémiques, traumatiques ou dégénératives du système nerveux central ou périphérique, les maladies inflammatoires aiguës ou chroniques et les maladies auto-immunes.

La présente invention a également pour objet les compositions pharmaceutiques contenant comme principe actif un dérivé de formule générale I ou un de ses sels physiologiquement tolérables, mélangé ou associé à un excipient pharmaceutique approprié, comme par exemple, le glucose, le lactose, l'amidon, le talc, l'éthyl cellulose, le stéarate de magnésium ou le beurre de cacao.

Les compositions pharmaceutiques ainsi obtenues se présentent généralement sous forme dosée et peuvent contenir de 1 à 200 mg de principe actif. Elles peuvent revêtir, par exemple, la forme de comprimés, dragées, gélules, suppositoires, solutions injectables ou buvables et être, selon les cas, administrées par voie orale, rectale ou parentérale à la dose de 1 à 200 mg 2 à 3 fois par jour.

Les exemples suivants illustrent la présente invention, les points de fusion étant déterminés à la platine chauffante de Kofler, sauf mention contraire.

**Exemple 1:**

(triméthoxy-2,3,4 benzyl) thio-4 cinnamyl-1 pipéridine :

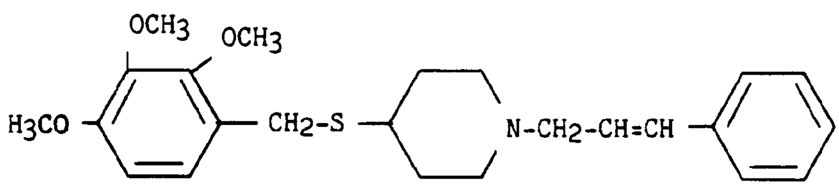

On chauffe, pendant 15 heures à reflux, un mélange de 10 g de (triméthoxy-2,3,4 benzyl)thio-4 pipéridine et 6,62 g de bromure de cinnamyle dans 300 ml de $CH_3CN$, en présence de 4,64 g de carbonate de potassium et 0,5 g d'iodure de potassium. On filtre ensuite le sel et évapore le filtrat sous pression réduite. Le résidu est repris par $CH_2Cl_2$-$H_2O$ et après décantation la phase dichlorométhane est séchée sur $Na_2SO_4$ et évaporée sous pression réduite.

Le résidu obtenu (15 g) est purifié par chromatographie sur 600 g de silice avec le système toluène-méthanol (95-5). On obtient 10 g de base brute, laquelle est transformée en fumarate en milieu éthanol. On obtient 7 g de fumarate de (triméthoxy-2,3,4 benzyl)thio-4 cinnamyl-1 pipéridine, sous forme de cristaux blancs fondant à 129°C.

La (triméthoxy-2,3,4 benzyl)thio-4 pipéridine de départ, dont le fumarate fond à 154°C, a été préparée par hydrolyse, au moyen de potasse et d'éthanol, de la (triméthoxy-2,3,4 benzyl)thio-4 carbéthoxy-1 pipéridine (huile), elle-même préparée par action de $ClCOOC_2H_5$ dans le benzène sur la (triméthoxy-2,3,4 benzyl)thio-4 méthyl-1 pipéridine (P.F. du fumarate correspondant : 130°C), elle-même préparée par condensation du chlorure de triméthoxy-2,3,4 benzyle dans l'éthanol sur le sel de sodium de la méthyl-1 mercapto-4 pipéridine, $Eb/_{13}$: 70-72°C.

De la même façon, ont été préparés les dérivés objet des exemples 2 à 30.

**Exemples 2-30 :**

**2)** La (triméthoxy-2,3,4 benzyl)thio-4 bis p.fluorobenzhydryl-1 pipéridine et son fumarate P.F. : 179°C (éthanol/éther).

**3)** La (triméthoxy-2,3,4 benzyl)thio-4 (benzofurannyl-2) méthyl-1 pipéridine, et son chlorhydrate P.F. (cap) : 165°C (éthanol).

**4)** La (triméthoxy-2,3,4 benzyl)thio-4 (fluoro-5 benzofurannyl-2) méthyl-1 pipéridine, et son chlorhydrate P.F. : 188°C. (éthanol).

**5)** La (triméthoxy-2,3,4 benzyl)thio-4 (benzothiényl-2) méthyl-1 pipéridine, P.F. : 115°C.

**6)** La cinnamylthio-4 (triméthoxy-2,3,4 benzyl)-1 pipéridine et son fumarate P.F. : 165°C. (éthanol).

**7)** La (triméthoxy-2,3,4 benzyl)thio-4 (fluoro-4 cinnamyl)-1 pipéridine et son chlorhydrate P.F. : 164°C. (éthanol/éther).

**8)** La (triméthoxy-3,4,5 benzyl)thio-4 cinnamyl-1 pipéridine, P.F. : 92°C.

**9)** La (triméthoxy-2,3,4 benzyl)thio-4 ($\Delta$3-chroményl-3) méthyl-1 pipéridine et son chlorhydrate P.F. : 202°C. (éthanol).

**10)** La (triméthoxy-2,4,5 benzyl)thio-4 cinnamyl-1 pipéridine, P.F. : 105°C.

**11)** La benzhydrylthio-4 (triméthoxy-2,3,4 benzyl)-1 pipéridine et son fumarate P.F. : 105°C. (éthanol).

**12)** La bis-p.fluorobenzhydrylthio-4 (triméthoxy-2,3,4 benzyl)-1 pipéridine et son fumarate P.F. (cap) : 170-172°C. (éthanol).

**13)** La (triméthoxy-2,3,4 benzyl)thio-4 (ditert.butyl-3,5 hydroxy-4 benzyl)-1 pipéridine et son fumarate P.F. : 202°C.

**14)** La (triméthoxy-2,3,4 benzyl)thio-4 [(ditert.butyl-3,5 hydroxy-4 phénoxy)-3 propyl]-1 pipéridine et son fumarate P.F. (cap) : 156-158°C.

**15)** La (triméthoxy-2,3,4 benzyl)thio-4 [(ditert.butyl-3,5 hydroxy-4 phényl)-3 propyl]-1 pipéridine et son fumarate P.F. (cap) : 143-145°C.

**16)** La (triméthoxy-2,3,4 benzyl)thio-4 [(ditert.butyl-3,5 hydroxy-4 phényl thio)-3 hydroxy-2 propyl]-1 pipéridine et son fumarate P.F. : 120°C.

**17)** La (triméthoxy-2,3,4 benzyl) thio-4 [(ditert.butyl-3,5 hydroxy-4 phénoxy)-3 hydroxy-2 propyl]-1 pipéridine et son fumarate P.F. : 160°C.

**18)** La (triméthoxy-2,3,4 benzyl)thio-4 [(ditert.butyl-3,5 hydroxy-4 phényl thio)-3 propyl]-1 pipéridine et son fumarate P.F. : 135°C.

**19)** La (triméthoxy-2,3,4 benzyl)thio-4 [(triméthoxy-2,3,5 hydroxy-4 phénoxy)-3 propyl]-1 pipéridine et son fumarate P.F. : 164°C.

**20)** La (triméthoxy-2,3,4 benzyl)thio-4 [(ditert.butyl-3,5 hydroxy-4 phénylthio)-5 pentyl]-1 pipéridine et son fumarate P.F. : 138°C.

**21)** La (triméthoxy-2,3,4 benzyl)thio-4 [(ditert.butyl-3,5 hydroxy-4 phénoxy)-5 pentyl]-1 pipéridine et son fumarate P.F. : 142°C.

**22)** La (triméthoxy-2,3,4 benzyl)thio-4 [(ditert.butyl-3,5 hydroxy-4 phénylthio)-3 méthyl-3 butyl]-1 pipéridine.

**23)** La triméthoxy-2,3,4 benzyl)thio-4 [(ditert.butyl-3,5 hydroxy-4 phénoxy)-3 méthyl-3 butyl]-1 pipéridine.

**24)** La (triméthoxy-2,3,4 benzyl)thio-4 [(triméthyl-2,3,5 hydroxy-4 phénoxy)-3 méthyl-3 butyl]-1 pipéridine.

**25)** La (triméthoxy-2,3,4 benzyl)thio-4 [(ditert.butyl-3,5 hydroxy-4 phényl-thio)-3 diméthyl-2,2 propyl]-1 pipéridine.

**26)** La (triméthoxy-2,3,4 benzyl)thio-4 [(ditert.butyl-3,5 hydroxy-4 phénoxy)-3 diméthyl-2,2 propyl]-1 pipéridine.

**27)** La (triméthoxy-2,3,4 benzyl)thio-4 [(triméthyl-2,3,5 hydroxy-4 phénoxy)-3 diméthyl-2,2 propyl]-1 pipéridine.

**28)** La (triméthoxy-2,3,4 benzyl)thio-4 [(méthyl-2 triméthyl-5,7,8 hydroxy-6 chromanyl-2)méthyl]-1 pipéridine.

**29)** La (triméthoxy-2,3,4 benzyl)thio-4 [(méthyl-2 triméthyl-5,7,8 hydroxy-6 chromanyl-2)-2 éthyl]-1 pipéridine.

**30)** La (triméthoxy-2,3,4 benzyl)thio-4 [(méthyl-2 triméthyl-5,7,8 hydroxy-6 chromanyl-2)-3 propyl]-1 pipéridine.

**Exemple 31:**

(triméthoxy-2,3,4 benzyl)thio-4 bis p.fluorobenzhydryl-1 pipéridine :

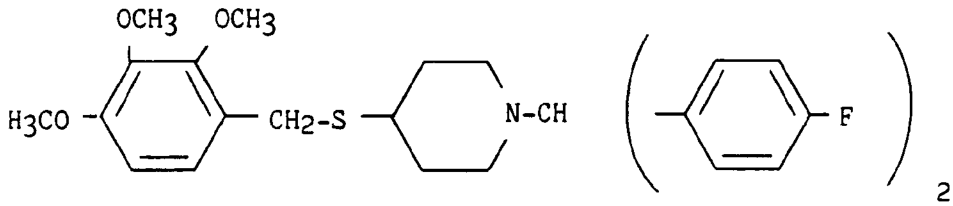

A une solution de 3,2 g de mercapto-4 bis p.fluorobenzhydryl-1 pipéridine dans 50 ml de diméthylformamide, on ajoute 0,5 g d'hydrure de sodium à 50% dans l'huile.

On agite pendant une heure et ajoute à la solution obtenue 2,4 g de chlorure de triméthoxy-2,3,4 benzyle. La solution est ensuite chauffée, pendant 3 heures, à 80°C, puis refroidie et le diméthylformamide est évaporé.

La phase résiduelle est traitée par l'eau et le dichlorométhane. On décante la phase dichlorométhane et après évaporation en obtient 5 g de base brute que l'on chromatographie sur 200 g de silice en éluant avec le mélange toluène-méthanol (95-5).

On recueille finalement 3,5 g de (triméthoxy-2,3,4 benzyl)thio-4 bis p.fluorobenzhydryl-1 pipéridine, sous forme de base pure, dont le fumarate cristallisé au sein de l'éthanol, fond à 179°C. La mercapto-4 bis p.fluorobenzhydryl-1 pipéridine de départ (huile) a été préparée par analogie avec la méthode de H. BARRERA et coll. J. Org. Chem. 27, 641-643 (1962) à partir de la benzhydryl-1 pipéridinone-4 fondant à 108°C.

Les matières premières (II) utilisées pour la synthèse des dérivés précédemment exemplifiés sont répertoriées dans le tableau ci-après :

Dérivés de formule :

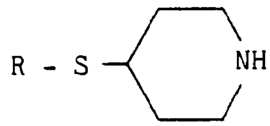

| R | P.F. °C (Kofler) |
|---|---|
| 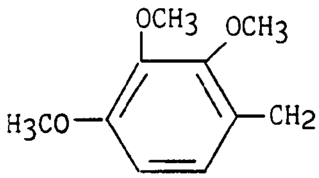 | 72 |
| 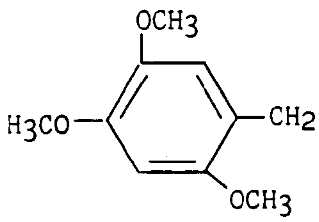 | huile |
| 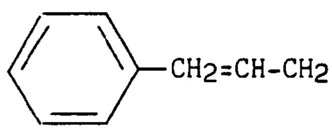 | ≃ 35 |
| 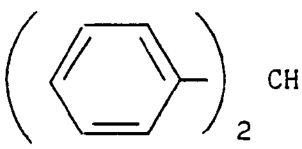 | 74 - 76 |

Les matières premières halogénées ou tosyloxy utilisées pour la synthèse des dérivés précédemment exemplifiés sont répertoriées dans le tableau ci-après.

| Dérivés | P.F. °C (Kofler) | Méthode de préparation |
|---|---|---|
| 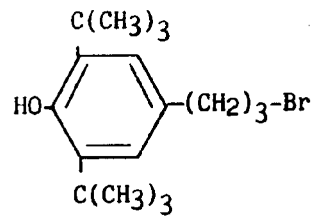 | 58 - 60 (cap) | Bromuration de l'alcool avec $(C_6H_5)_3$ P Br dans DMF |
| 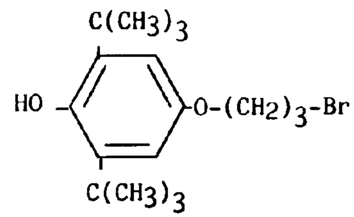 | 35 - 36 | 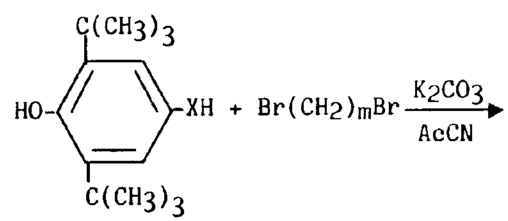 |
| 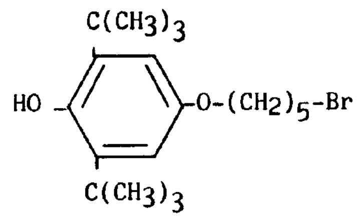 | Huile | |
| 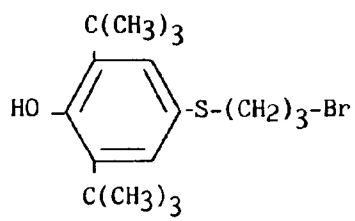 | Huile | |

| Dérivés | P.F. °C (Kofler) | Méthode de préparation |
|---|---|---|
| 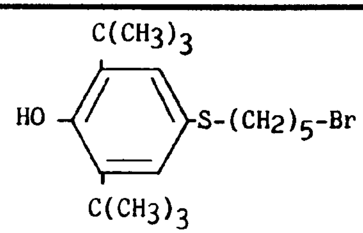 | Huile | 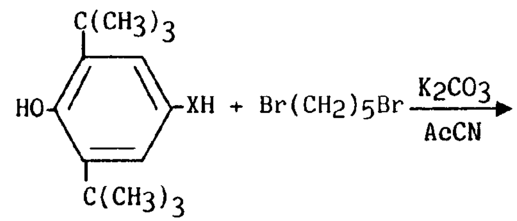 |
| 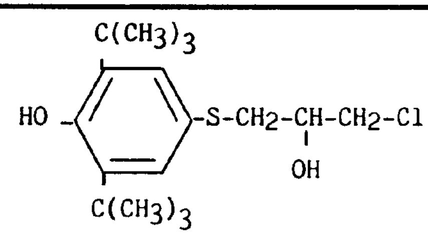 | 68 | |
| 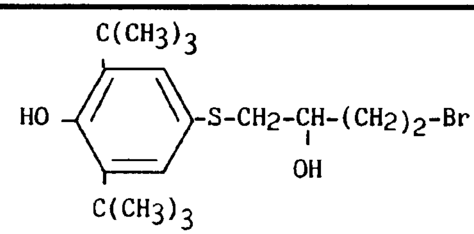 | 125 | |
| 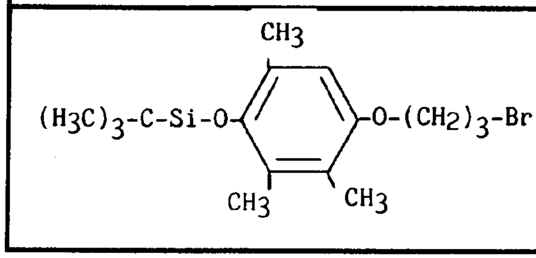 | 45 - 46 | Selon T. YOSMIOKA et coll. J. méd. Chem.32, 421-428 (1989) |

| Dérivés | P.F. °C (Kofler) | Méthode de préparation |
|---|---|---|
| 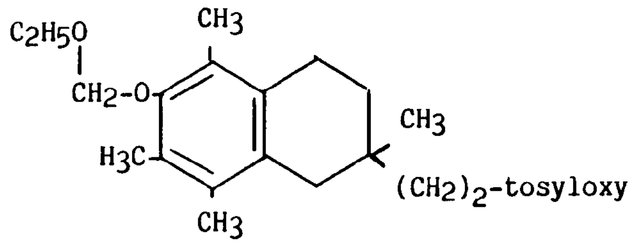 | Huile | Tosylation de l'alcool préparé selon J. Scott et coll., J. am. oil. Chem Soc. 51, (5), 200 - 203, (1974) |
| 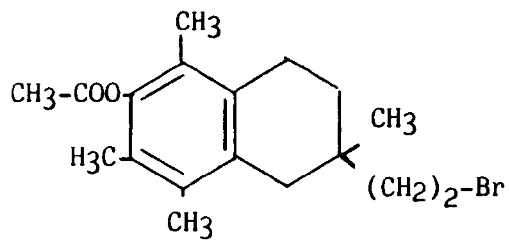 | 102 - 103 | Selon MERREL E.P. 369.874 |
| 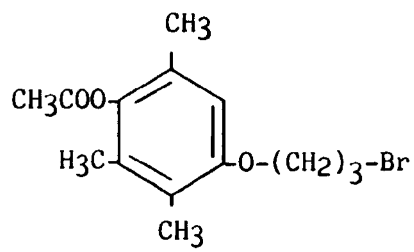 | 54 | 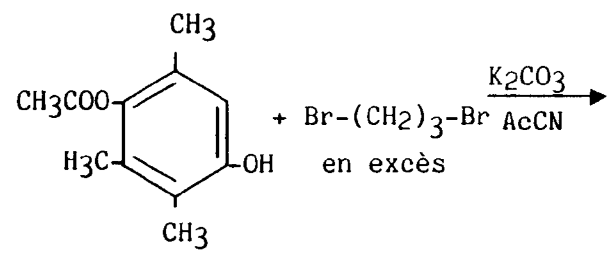  |

**EXEMPLE 32:**

**ETUDE PHARMACOLOGIQUE :**

**A) ETUDE IN VITRO :**

**I. PROTECTION VIS A VIS DE L'ACIDOSE INTRACELLULAIRE ET INHIBITION DES CANAUX CALCIQUES LENTS :**

L'action des dérivés de la présente invention et en particulier du dérivé objet de l'exemple 1, a été démontrée sur des cellules cardiaques de rat. Les effets protecteurs cellulaires à l'encontre des événements associés à l'acidose intracellulaire (ischémie-anoxie) ainsi que l'effet inhibiteur sur l'entrée du calcium par les canaux calcium lents, ont été démontrés. Les effets protecteurs du dérivé de l'exemple 1 ont été évalués vis-à-vis de la séquence primaire des événements nécrosants induits lors de l'ischémie et cela de façon comparative à ceux des dichlorhydrate de trimétazidine (DCI) et de la cinnarizine (DCI) pris comme produit de référence.

**α) Matériels et méthodes**

Culture de cellules

**a)** Cellules cardiaques : les cellules cardiaques sont préparées à partir de coeur de rats Wistar nouveau nés (3 jours) selon la technique de RENAUD JF, MEAUX JP, ROMEY G, SCHMID A, LAZDUNSKI M, Activation of the voltage-dependent $Ca^{2+}$ channels in rat heart cells by dihydropyridine derivative-Biochem. Biophys. Res. Commun., 125, 405-412, (1984).

Les cardiomyocytes sont isolés par dissociation enzymatique des ventricules et ensemencés à 185.000 cellules/$cm^2$. Les cellules sont maintenues à 37°C sous atmosphère air/$CO_2$ (95V/5V) saturée en eau. Les cardiomyocytes sont utilisés entre le 3ème et le 8ème jour après la mise en culture.

**b)** $A_7r_5$ : lignées cellulaires provenant d'aorte de rats nouveau-nés (ATCC n°CRL 1444) cultivées dans les conditions indiquées par l'American Type Culture Collection (ATCC).

Etude de liaison

Les études de liaison sont effectuées sur des fractions microsomales enrichies en canaux calcium de type L [cf. FOSSET M., LAZDUNSKI M. Biochemical characterization of the skeletal muscle $Ca^{2+}$ channel. In : Receptors Biochemistry and Methodology Venter JC, Triggle D., eds vol. 9, 141-159 (1986) et O'BRIEN R.A. Receptor binding in drug research. In : clinical pharmacology vol. 5. (1986)].

Les expériences de compétition entre les différents radioligands et les molécules de référence sont conduites dans les conditions standards de liaison selon O'BRIEN R.A. Receptor binding in drug research. In : clinical pharmacology vol. 5 DEKKER M. Inc., New York and Basel, (1986). Dans tous les cas, après le temps requis d'association, la radiocativité liée est séparée de la composante libre par filtration sous vide sur filtre GF/C.

Flux ioniques

Les influx de $^{45}ca^{2+}$ sont évalués sur les $A_7r_5$ en présence ou absence de D 888 (inhibiteur spécifique du canal $Ca^{2+}$ de type L) et en présence ou absence de concentrations variables du dérivé de l'exemple 1 dans les conditions publiées sur le coeur par RENAUD JF., KAZASOGLOU T., SCHMID A., ROMEY G., LAZDUNSKI M. Differentiation of [3H] nitrendipine receptor sites in chick hearts and physiological relation to the slow $Ca^{2+}$ channel and to excitation-contraction coupling. Eur. J. Biochem., 139 673-681, (1984).

Electrophysiologie

Les études ont été réalisées sur la lignée cellulaire $A_7r_5$ en utilisant la technique de "whole cell patch-clamp" décrite par HAMILL O., MARTY A., WEHER E., SAKMANN B., SIGWORTH F.F. Improved patch clamp techniques for high resolution current recording from cells and cell from membrane patches. Pflügers Arch., 391, 85-100, (1981).

mesure de l'indice de protection après induction d'une acidose intracellulaire

L'acidose intracellulaire est induite selon les conditions publiées par RENAUD JF. Internal pH $Na^+$ and $Ca^{2+}$ regulation by trimetazidine during cardiac cell acidosis. Cardiovasc. Drugs and Ther., 1, 667-686, (1988) en présence ou absence des produits à tester. La nécrose cellulaire est évaluée en mesurant la cinétique de libération dans le milieu extracellulaire d'un enzyme cytosolique l'α-hydroxybutyrate-deshydrogénase (αHBDH) par spectrophotométrie en utilisant le kit Boehringer 124800.

β) **Résultats**

**1)** Effet du dérivé de l'exemple 1 sur la fixation du PN 200-110 et du D888 au niveau du canal $Ca^{2+}$. Comparaison avec le dichlorhydrate de trimétazidine et la cinnarizine.

| | Référence $K_{0,5}$(M) | Dérivé de l'exemple 1 $K_{0,5}$(M) | Trimétazidine, 2 HCl $K_{0,5}$(M) | Cinnarizine $K_{0,5}$(M) |
|---|---|---|---|---|
| [3H] PN 200-110 | Nifédipine 2,5 $10^{-8}$ | $10^{-6}$ | $>10^{-4}$ | 3,4 $10^{-7}$ |
| [3H] D888 | D888 2,5 $10^{-9}$ | 1,3 $10^{-6}$ | $>10^{-4}$ | 5,2 $10^{-7}$ |

Les valeurs de K0,5 sont exprimées en Molaire (mole/l) et correspondent à la concentration pour laquelle 50% de la radioactivité fixée de façon spécifique est déplacée par le produit considéré.

**2)** Effet du dérivé de l'exemple 1 sur l'inhibition de l'influx de $Ca^{2+}$ passant par le canal $Ca^{2+}$. Comparaison avec le dichlorhydrate de trimétazidine et la cinnarizine.

| | Référence $K_{0,5}$(M) | Dérivé de l'exemple 1 $K_{0,5}$(M) | Trimétazidine, 2 HCl $K_{0,5}$(M) | Cinnarizine $K_{0,5}$(M) |
|---|---|---|---|---|
| D888 | 2,3 $10^{-8}$ | 3 $10^{-6}$ | $>10^{-4}$ | 1,2 $10^{-7}$ |

Les valeurs de K0,5 sont exprimées en Molaire (mole/1) et correspondent à la concentration pour laquelle 50% de l'influx de $^{45}Ca^{2+}$ passant par le canal $Ca^{2+}$ est inhibé par le produit considéré.

**3)** Effet du dérivé de l'exemple 1 sur les récepteurs de neurotransmetteurs. Comparaison avec le dichlorhydrate de trimétazidine. (cf. tableau ci-après).

| Récepteurs | Ligand | Référence | $K_{0,5}$ (M) | Dérivé de l'exemple 1 $K_{0,5}$ (M) | Trimétazidine 2 HCl | Cinnarizine $K_{0,5}$ (M) |
|---|---|---|---|---|---|---|
| Sérotonine | | | | | | |
| $5HT_{1A}$ | [3H] 8 OH DPAT | 8 OH DPAT | 3 $10^{-9}$ | 3 $10^{-6}$ | » $10^{-4}$ | 4 $10^{-6}$ |
| $5HT_{1B}$ | [125] CYP | Sérotonine | 3,5 $10^{-8}$ | $10^{-5}$ | $10^{-4}$ | $10^{-4}$ |
| $5HT_2$ | [3H] Kétansérine | Kétansérine | 8,2 $10^{-9}$ | $10^{-6}$ | > $10^{-4}$ | $10^{-6}$ |
| Alpha adrénergique | | | | | | |
| α1 | [3H] Prazosin | Prazosin | 6,$10^{-10}$ | 2 $10^{-6}$ | / | 4 $10^{-7}$ |
| α2 | [3H] Rauwolscine | Yohimbine | 3,1 $10^{-8}$ | 9 $10^{-7}$ | / | 2 $10^{-6}$ |
| Bêta adrénergique | | | | | | |
| β | [3H] DHA | Alprénolol | 2,1 $10^{-9}$ | 2 $10^{-6}$ | > $10^{-4}$ | > $10^{-4}$ |
| Dopamine | | | | | | |
| $D_1$ | [3H] SCH 23390 | SCH 23390 | 7 $10^{-10}$ | 3 $10^{-7}$ | / | 3 $10^{-7}$ |
| $D_2$ | [3H] 205 501 | Butaclamol | $10^{-9}$ | 3 $10^{-7}$ | / | $10^{-7}$ |

**4)** Effet protecteur du dérivé de l'exemple 1 sur les conséquences associées à une acidose intracellulaire. Comparaison avec le dichlorhydrate de trimétazidine.

| Concentrations µ M | 0.3 | 1 | 3 | 10 | 20 | 30 | 100 | 500 |
|---|---|---|---|---|---|---|---|---|
| Dérivé de l'exemple 1 | 143,99 ± 26,72 (n = 4) | 21,95 ± 1,19 (n = 4) | 1,42 ± 0,74 (n = 4) | 2,14 ± 0,8 (n = 4) | / | 9,68 ± 2,69 (n = 4) | / | / |
| Trimétazidine 2 HCl | / | / | / | / | 94,58 ± 12,53 (n = 3) | / | 75,76 ± 7,79 (n = 3) | 58,26 ± 8,13 (n = 3) |
| contrôle avant acidose | 0 % ± 0,86 n=3 | | | | | | | |
| contrôle après acidose seule | 100 % ± 11,10 n=4 | | | | | | | |

Le signal mesuré correspond à l'amplitude de la nécrose induite par l'acidose. Les valeurs sont les moyennes ±sem. Le nombre d'expériences est indiqué entre parenthèses.

**5)** Etudes électrophysiologiques réalisées à partir d'une lignée de cellules aortiques de rat ($A_7r_5$).

Les études montrent, dans les conditions de whole cell patch clamp, une inhibition du courant entrant calcique lent avec un demi-effet ($EC_{50}$) pour le dérivé de l'exemple 1 à $10^{-5}$M.

**6)** En conclusion :

Les résultats obtenus en 1, 2 et 5 montrent, que le dérivé de l'exemple 1 s'associe aux récepteurs du canal $Ca^{2+}$ lent et que cette association induit d'une part, l'inhibition de l'influx de $Ca^{2+}$ et d'autre part, l'inhibition du courant entrant $Ca^{2+}$ de façon dose-dépendante.

Ces résultats différencient le dérivé de l'exemple 1 du dichlorhydrate de trimétazidine par ses propriétés anticalciques et l'en rapproche de la cinnarizine avec une bonne relation dose-effet. Les résultats présentés en 3 montrent que le dérivé de l'exemple 1 intéragit de façon peu spécifique et avec une faible affinité au niveau des récepteurs de neurotransmetteurs (5HT, α, β) à l'exception des récepteurs dopaminergiques. Les résultats présentés en 4 montrent que le dérivé de l'exemple 1 protège la cellule cardiaque de la nécrose induite par l'acidose intracellulaire. Cette protection a lieu dès 1 µM pour être maximum entre 3 et 10 µM de dérivé de l'exemple 1 avec 99 à 98 % de protection. Dans les mêmes conditions, le dichlorhydrate de trimétazidine, pour une concentration 500 fois supérieure (500 µM), ne protège que 42 % de la population cellulaire soumise à l'acidose intracellulaire.

## II. PROTECTION VIS A VIS DES MODIFICATIONS OXYDATIVES DES LDL ET DE LA NECROSE OXYDATIVE DE CELLULES CARDIAQUES.

L'activite des composés de la présente invention a été mise en évidence in vitro sur la modification oxydative des LDL humaines induite par le sulfate de cuivre, et sur la nécrose des cellules cardiaques.

**α) Matériels et methodes**

a)Modification des LDL par le sulfate de cuivre

Les LDL humaines sont incubées 24 heures en présence de sulfate de cuivre (5 $10^{-6}$ M) et en absence ou en présence des composés testés ($10^{-7}$M à $10^{-4}$ M).

Après incubation, la peroxydation des LDL est évaluée par électrophorèse sur gel d'agar et par la formation

de l'un des produits de la peroxydation lipidique: le malondialdéhyde (MDA) (Parthasarathy S., Young S.G., Witztum J.L., Pittman R.C., et Steinberg D.; J. Clin. Invest. 77; 641-644, 1986).
L'activité des composés testés est évaluée par le calcul des concentrations réduisant de 50 % (IC50) la production de MDA par rapport aux expériences contrôles en absence de produit.

b)Nécrose oxydative des cellules cardiaques

Les cellules cardiaques de rats nouveau-nés sont utilisées entre les 5ème et 7ème jours après la mise en culture. La nécrose oxydative est induite par le système enzymatique producteur de radicaux libres hypoxanthine (HX, 1mM) et xanthine-oxydase (XO, 10mU/ml). La nécrose est évaluée 4 heures après l'adjonction de XO/HX en mesurant spectrophotométriquement l'activité cytosolique $\alpha$-hydroxy-butyrate deshydrogenase ($\alpha$-HBDH) libérée dans le surnageant. Deux molécules de référence (probucol, vitamine E) et 2 molécules représentatives de la présente invention (correspondant aux exemples 13 et 19) ont été testées. Les cellules sont traitées avec les molécules 16 heures et 1 heure avant le début de l'expérimentation après renouvellement du milieu. En début d'expérience, le traitement est renouvelé une dernière fois.

**β)résultats**

1)Effet sur la modification des LDL :

Le tableau ci-après rassemble les IC50, appréciant le pouvoir initiateur de la peroxydation lipidique des LDL humaines, obtenues avec un échantillon des dérivés de l'invention et les produits antioxydants de référence: probucol et vitamine E, sur le test d'oxydation des LDL par le sulfate de cuivre ($Cu^{2+}$)

| Composés | IC50(M) (Cu2+) |
|---|---|
| Exemple 13 | 3 $10^{-6}$ |
| Exemple 14 | $10^{-6}$ |
| Exemple 15 | 3 $10^{-6}$ |
| Exemple 16 | 3 $10^{-6}$ |
| Exemple 17 | 2 $10^{-6}$ |
| Exemple 18 | 2 $10^{-6}$ |
| Exemple 19 | 3 $10^{-7}$ |
| Exemple 20 | $10^{-6}$ |
| Exemple 21 | 8 $10^{-7}$ |
| Probucol | 3 $10^{-6}$ |
| Vitamine E | $>10^{-4}$ |

Ces résultats indiquent clairement la plus grande puissance notamment du composé de l'exemple 19 par rapport au probucol ou à la vitamine E à protéger les LDL humaines vis à vis des modifications induites par le sulfate de cuivre.

2) Effet sur la nécrose oxydative des cellules cardiaques :

Le tableau suivant regroupe les index de nécrose de cellules cardiaques induites par le système hypoxanthine/xantine oxydase seul ou en présence de concentrations croissantes des composés de l'invention testés ou des produits de référence : probucol et vitamine E.

| Composés | Contrôle XO + HX | $10^{-7}$ M XO + HX | $10^{-6}$ M XO + HX | $10^{-5}$ M XO + HX |
|---|---|---|---|---|
| Exemple 13 | 100,0±19,2 | 75,7± 19,7 | 52,4±18,2 | 16,1±5,6 |
| Exemple 19 | 100,0±6.8 | 86,0±11,5 | 38,4±13,7 | 7,9±4,1 |
| Probucol | 100,0±11.9 | | 95,0±1,7 | 68,0±0,9 |
| Vitamine E | 100,0±10,6 | 97,1±2,6 | 87,6±5,7 | 54,9±11,3 |

L'index de nécrose 100 correspond à une libération de 46,7±5,3% en 4 heures du contenu cytosolique en α-HBDH dans le surnageant.

Les résultats sont exprimés sous forme de moyenne ± sem (2< n ≦4, 3 à 4 répétitions par expérience).

Les composés des exemples 13 et 19 qui réduisent de 50% ou plus la nécrose oxydative des cardiomyocytes dès $10^{-6}$M sont nettement supérieurs aux produits antioxydants de référence dont l'activité, plus faible, n'apparait qu'à $10^{-5}$ M.

**B) ETUDE IN VIVO :**

MISE EN EVIDENCE DE L'ACTIVITE ANTIISCHEMIQUE

**1)** METHODOLOGIE

Les études sont réalisées sur des chiens beagle de 9 à 12 kg, anesthésiés au pentobarbital sodique 30 mg/kg iv, intubés et ventilés, soumis à une thoracotomie au 5ème espace intercostal gauche.

Une bague électromagnétique reliée à un débimètre électromagnétique Gould SP 2202 est placée autour de la branche circonflexe de l'artère coronaire gauche, un ballonnet gonflable est également placé juste en aval de cette bague pour la réalisation des sténoses coronaires sous contrôle du débit coronaire.

Des cristaux piezoélectriques (5 MHz) reliés à un sonomicromètre Triton sont implantés dans le sous endocarde de la paroi ventriculaire gauche, dans une zone devant devenir ischémique, pour la mesure de la contractilité régionale myocardique ; dans cette même zone des électrodes épicardiques sont placées pour le recueil de l'électrocardiogramme épicardique.

Deux électrodes sont cousues au niveau de l'oreillette droite dans la région du noeud sinusal pour les protocoles incluant des séquences de pacing.

**2)** PROTOCOLES EXPERIMENTAUX

Deux types de protocole d'ischémie myocardique sont réalisés :

Protocole 1

L'ischémie myocardique est induite par une sténose coronaire serrée réduisant d'environ 70 % le débit coronaire.

Deux périodes de 10 minutes de cette même sténose coronaire, aux effets reversibles et reproductibles, sont réalisées chez le même animal, séparées par un intervalle de récupération de 45 minutes.

Les traitements sont administrés par voie intraveineuse 2 minutes après sténose coronaire :

- injection de solvant après la première sténose coronaire qui sert de contrôle,
- injection de produit testé après la seconde sténose coronaire.

Protocole 2

Une sténose coronaire modérée, réduisant le débit coronaire de 30 à 50%, insuffisante à elle seule pour provoquer une ischémie, est établie.

L'ischémie myocardique est induite par accélération de la fréquence cardiaque d'environ 90 battements par minute, par pacing auriculaire maintenu pendant 2 minutes.

Deux séquences de pacing aux effets réversibles et reproductibles, sont réalisées chez le même animal,

une première précédée de l'injection de solvant sert de témoin, une seconde après 45 minutes de récupération est précédée par l'injection du produit à tester.

Les traitements sont administrés par voie intraveineuse 3 minutes avant la séquence de pacing.

3) PARAMETRES MESURES

La contractilité myocardique est appréciée par la fraction de raccourcissement systolique des longueurs segmentaires comprises entre les cristaux piezoélectriques.

L'hypokinésie en zone ischémique est exprimée en pourcentage de variation par rapport à la contractilité mesurée en période contrôle.

La modification de l'ECG épicardique en zone ischémique consiste en une surélévation du segment ST épicardique mesurée en millivolt (mV).

4) RESULTATS

Protocole 1

| Paramètres | Traitement | Sténose | 10 minutes après traitement |
|---|---|---|---|
| Contractilité delta % | Solvant | - 45,8 | - 44,5 |
| | dérivé de l'exemple 1 1 mg/kg iv | - 45 | - 24,3 |
| ST épicardique delta mV | Solvant | + 4,5 | + 4 |
| | dérivé de l'exemple 1 1 mg/kg | + 4,5 | + 2 |

Protocole 2

| Paramètres | Traitement | Pacing 2 minutes |
|---|---|---|
| Contractilité delta % | Solvant | - 62.6 |
| | dérivé de l'exemple 1 1 mg/kg iv | - 42,2 |
| ST épicardique delta mV | Solvant | + 6,0 |
| | dérivé de l'exemple 1 1 mg/kg iv | + 0,5 |

En conclusion :

Administré par voie intraveineuse, le dérivé de l'exemple 1 réduit nettement les modifications électrocardiographiques et de la contractilité myocardique induites par ischémie tant en préventif qu'en curatif.

## Revendications

### Revendications pour les Etats contractants suivants : AT, BE, CH, DE, DK, FR, GB, IT, LI, LU, NL, SE

**1.** Les dérivés de la pipéridine de formule générale I :

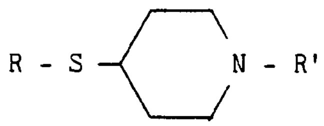

(I)

dans laquelle :

- l'un des substituants R ou R' représente le radical de formule

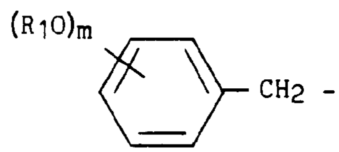

dans laquelle :

- m est un nombre entier de 1 à 3 et
- $R_1$ représente un radical alkyle ayant de 1 à 5 atomes de carbone,

et

- l'autre substituant R ou R' représente :

soit un radical de formule :

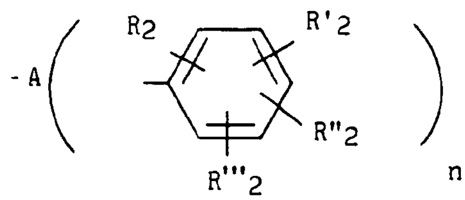

dans laquelle

- A représente une chaîne hydrocarbonée ayant de 1 à 5 atomes de carbone renfermant éventuellement une double liaison ou un atome d'oxygène ou de soufre, éventuellement substituée par un radical hydroxy ou un ou plusieurs radicaux méthyle ;
- n prend les valeurs 1 ou 2 ;

et

$R_2$, $R'_2$, $R''_2$ et $R'''_2$, identiques ou différents, représentent chacun un atome d'hydrogène, un atome d'halogène tel qu'un atome de chlore ou de fluor, un radical alkyl ou alkoxy ayant chacun de 1 à 5 atomes de carbone en chaîne droite ou ramifiée, ou un radical hydroxy ;

soit un radical de formule :

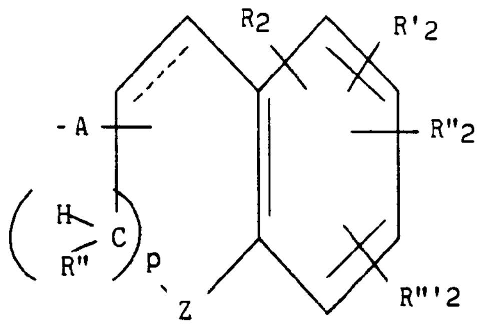

dans laquelle :

- Z représente un atome d'oxygène ou de soufre ;
- R" représente un atome d'hydrogène ou un radical méthyle ;
- p prend les valeurs zéro ou un ;

et

- A, $R_2$, $R'_2$, $R''_2$ et $R'''_2$ ont les significations précédemment définies.

**2.** Les sels physiologiquement tolérables des composés de la revendication 1 avec des acides appropriés.

**3.** La (triméthoxy-2,3,4 benzyl)thio-4 cinnamyl-1 pipéridine.

**4.** La (triméthoxy-2,3,4 benzyl)thio-4 (ditert.butyl-3,5 hydroxy-4 benzyl)-1 pipéridine et son fumarate.

**5.** La (triméthoxy-2,3,4 benzyl)thio-4[(triméthoxy-2,3,5 hydroxy-4 phénoxy)-3 propyl]-1 pipéridine et son fumarate.

**6.** Le procédé de préparation des dérivés de la revendication 1 caractérisé en ce que l'on condense :
- un dérivé de formule générale II :

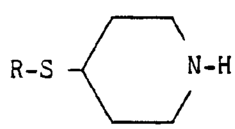

(II)

dans laquelle R a la signification définie dans la revendication 1,
- avec un dérivé de formule générale III :

Y-R' (III)

dans laquelle R' a la signification définie dans la revendication 1 et Y est un atome de chlore ou de brome, ou un radical tosyloxy.

**7.** Le procédé de préparation selon la revendication 6 caractérise en ce que l'on effectue la condensation des dérivés II et III dans un solvant polaire, en présence d'un accepteur de l'hydracide formé au cours de la réaction, à une température comprise entre 80 et 130°C.

**8.** Le procédé de préparation des dérivés de la revendication 1, caractérisé en ce que l'on condense un dérivé de formule générale IIa :

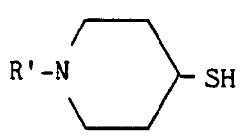

(IIa)

dans laquelle R' a la signification définie dans la revendication 1, avec un dérivé de formule générale IIIa :

Y-R (IIIa)

dans laquelle R a la signification définie dans la revendication 1 et Y a la signification définie dans la revendication 6.

9. Le procédé de préparation selon la revendication 8 caractérisé en ce que l'on effectue la condensation des dérivés IIa et IIIa, dans un solvant polaire, à une température comprise entre 80 et 110°C, en présence d'un agent alcalin.

10. Le procédé de préparation selon la revendication 8 caractérisé en ce que l'on effectue la condensation à partir d'un dérivé IIa préalablement sodé.

11. Les compositions pharmaceutiques contenant comme principe actif un dérivé selon les revendications 1 à 5 avec des excipients pharmaceutiques appropriés.

12. Les compositions pharmaceutiques selon la revendication 11, présentées sous une forme convenant notamment pour le traitement des pathologies liées à l'ischémie tissulaire et de la pathologie vasculaire périphérique, ou le traitement des pathologies dans lesquelles une peroxydation lipidique joue un rôle initiateur et/ou aggravant.

**Revendications pour les Etats contractants suivants : ES, GR**

1. Le procédé de préparation des dérivés de la pipéridine de formule générale I :

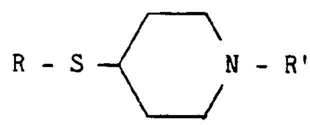

(I)

dans laquelle :
- l'un des substituants R ou R' représente le radical de formule

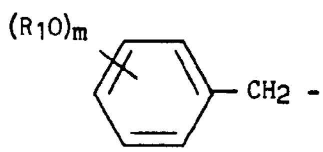

dans laquelle :
- m est un nombre entier de 1 à 3 et
- $R_1$ représente un radical alkyle ayant de 1 à 5 atomes de carbone,

et
- l'autre substituant R ou R' représente :
soit un radical de formule :

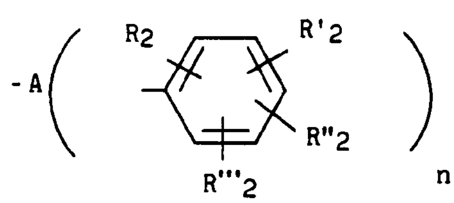

dans laquelle
- A représente une chaîne hydrocarbonée ayant de 1 à 5 atomes de carbone renfermant éventuellement une double liaison ou un atome d'oxygène ou de soufre, éventuellement substituée par un radical hydroxy ou un ou plusieurs radicaux méthyle ;
- n prend les valeurs 1 ou 2 ;

et

$R_2$, $R'_2$, $R''_2$ et $R'''_2$, identiques ou différents, représentent chacun un atome d'hydrogène, un atome d'halogène tel qu'un atome de chlore ou de fluor, un radical alkyl ou alkoxy ayant chacun de 1 à 5 atomes de carbone en chaîne droite ou ramifiée, ou un radical hydroxy ;
soit un radical de formule :

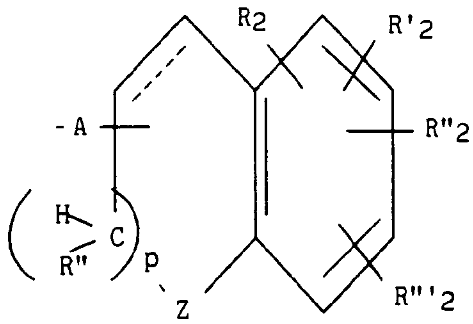

dans laquelle :
- Z représente un atome d'oxygène ou de soufre ;
- R" représente un atome d'hydrogène ou un radical méthyle ;
- p prend les valeurs zéro ou un ;

et
- A, $R_2$, $R'_2$, $R''_2$ et $R'''_2$ ont les significations précédemment définies, et de leurs sels physiologiquement tolérables avec des acides appropriés;

caractérisé en ce que l'on condense :
- un dérivé de formule générale II :

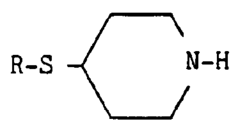 (II)

dans laquelle R a la signification précédemment définie
- avec un composé de formule générale III :

Y-R' (III)

dans laquelle R' a la signification précédemment définie et Y est un atome de chlore ou de brome, ou un radical tosyloxy.

2. Le procédé de préparation selon la revendication 1 caractérisé en ce que l'on effectue la condensation des dérivés II et III dans un solvant polaire, en présence d'un accepteur de l'hydracide formé au cours de la réaction, à une température comprise entre 80 et 130°C.

3. Le procédé de préparation des dérivés de formule générale I, définie dans la revendication 1, caractérisé en ce que l'on condense un dérivé de formule générale IIa :

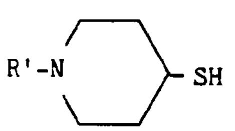 (IIa)

dans laquelle R' a la signification définie dans la revendication 1, avec un composé de formule générale IIIa :

Y-R (IIIa)

dans laquelle R et Y ont les significations définies dans la revendication 1 .

4. Le procédé de préparation selon la revendication 3 caractérisé en ce que l'on effectue la condensation

des dérivés IIa et IIIa, dans un solvant polaire, à une température comprise entre 80 et 110°C, en présence d'un agent alcalin.

5. Le procédé de préparation selon la revendication 3 caractérisé en ce que l'on effectue la condensation à partir d'un dérivé IIa préalablement sodé.

**Claims**

**Claims for the following Contracting States : AT, BE, CH, DE, DK, FR, GB, IT, LI, LU, NL, SE**

1. Piperidine compounds of the general formula I :

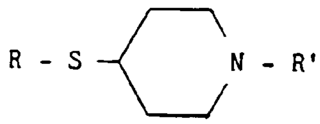

(I)

wherein :
- one of the substituents R or R' represents a radical of formula

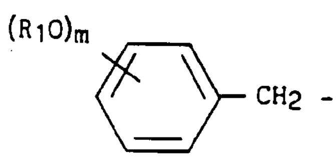

in which :
- m is an integer from 1 to 3 and
- $R_1$ represents an alkyl radical having from 1 to 5 carbon atoms, and
- the other substituent R or R' represents : either a radical of formula :

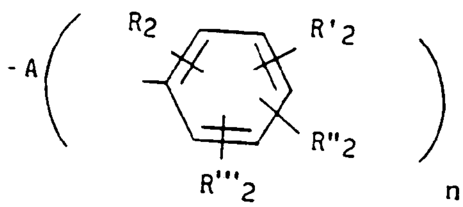

in which
- A represents a hydrocarbon chain having from 1 to 5 carbon atoms optionally containing a double bond or an oxygen or sulphur atom and optionally substituted by a hydroxy radical or by one or more methyl radicals;
- n is 1 or 2;

and
$R_2$, $R'_2$, $R''_2$ and $R'''_2$, which are the same or different, each represents a hydrogen atom, a halogen atom, such as a chlorine or fluorine atom, an alkyl or alkoxy radical each having from 1 to 5 carbon atoms in a straight or branched chain, or a hydroxy radical;
or a radical of formula :

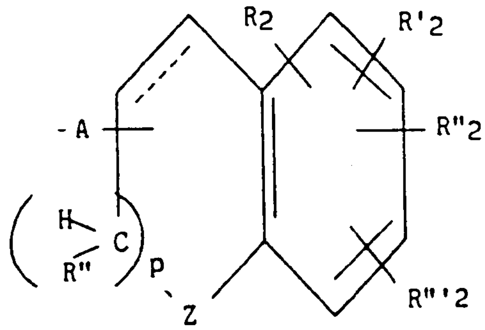

in which :
- Z represents an oxygen or sulphur atom;
- R" represents a hydrogen atom or a methyl radical;
- p is zero or one;

and
- A, $R_2$, $R'_2$, $R''_2$ and $R'''_2$ are as defined above.

2. The physiologically acceptable salts of compounds of claim 1 with appropriate acids.

3. 4-(2,3,4-trimethoxybenzyl)thio-1-cinnamyl-piperidine,

4. 4-(2,3,4-trimethoxybenzyl)thio-1-(3,5-ditert.-butyl-4-hydroxybenzyl)piperidine and its fumarate.

5. 4-(2,3,4-trimethoxybenzyl)thio-1-[3-(2,3,5-trimethoxy-4-hydroxyphenoxy)propyl]piperidine and its fumarate.

6. A process for the preparation of compounds according to claim 1, characterised in that :
- a compound of the general formula II :

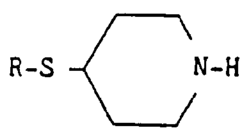

(II)

in which R is as defined in claim 1, is condensed
- with a compound of the general formula III :

Y-R' (III)

in which R' is as defined in claim 1 and Y is a chlorine or bromine atom or a tosyloxy radical.

7. A preparation process according to claim 6, characterised in that the condensation of compounds II and III is carried out in a polar solvent, at a temperature of from 80 to 130°C, in the presence of an acceptor of the hydracid formed during the course of the reaction.

8. A process for the preparation of compounds according to claim 1, characterised in that a compound of the general formula IIa :

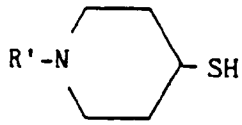

(IIa)

in which R' is as defined in claim 1, is condensed with a compound of the general formula IIIa :

Y - R (IIIa)

in which R is as defined in claim 1 and Y is as defined in claim 6.

9. A preparation process according to claim 8, characterised in that the condensation of compounds IIa and

IIa is carried out in a polar solvent at a temperature of from 80 to 110°C in the presence of an alkaline agent.

**10.** A preparation process according to claim 8, characterised in that the condensation is carried out starting from a compound IIa which has previously been sodified.

**11.** Pharmaceutical compositions comprising as active ingredient a compound according to any one of claims 1 to 5 together with appropriate pharmaceutical excipients.

**12.** Pharmaceutical compositions according to claim 11, presented in a form suitable especially for the treatment of pathologies associated with tissular ischaemia and peripheral vascular pathology or for the treatment of pathologies in which lipid peroxidation plays an initiating and/or aggravating role.

**Claims for the following Contracting States : ES, GR**

**1.** A process for the preparation of piperidine compounds of the general formula I :

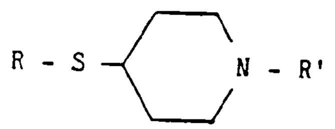

(I)

wherein :
- one of the substituents R or R' represents a radical of formula

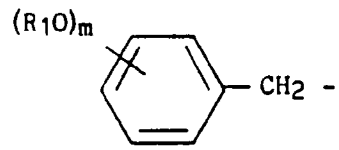

in which :
- m is an integer from 1 to 3 and
- $R_1$ represents an alkyl radical having from 1 to 5 carbon atoms, and
- the other substituent R or R' represents : either a radical of formula :

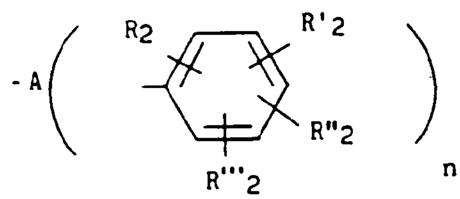

in which
- A represents a hydrocarbon chain having from 1 to 5 carbon atoms optionally containing a double bond or an oxygen or sulphur atom and optionally substituted by a hydroxy radical or by one or more methyl radicals;
- n is 1 or 2;
  and
  $R_2$, $R'_2$, $R''_2$ and $R'''_2$, which are the same or different, each represents a hydrogen atom, a halogen atom, such as a chlorine or fluorine atom, an alkyl or alkoxy radical each having from 1 to 5 carbon atoms in a straight or branched chain, or a hydroxy radical;

or a radical of formula :

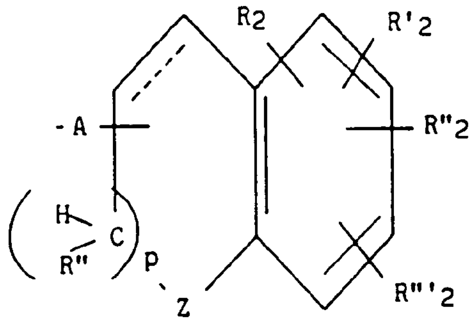

in which :

- Z represents an oxygen or sulphur atom;
- R" represents a hydrogen atom or a methyl radical;
- p is zero or one;

and

- A, $R_2$, $R'_2$, $R''_2$ and $R'''_2$ are as defined above, and the physiologically acceptable salts thereof with appropriate acids, characterised in that :
- a compound of the general formula II :

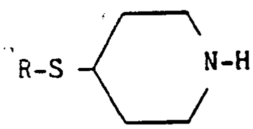

(II)

in which R is as defined above, is condensed

- with a compound of the general formula III :

Y-R' (III)

in which R' is as defined above and Y is a chlorine or bromine atom or a tosyloxy radical.

**2.** A preparation process according to claim 1, characterised in that the condensation of compounds II and III is carried out in a polar solvent, at a temperature of from 80 to 130°C, in the presence of an acceptor of the hydracid formed during the course of the reaction.

**3.** A process for the preparation of compounds according to the general formula I, defined in claim 1, characterised in that a compound of the general formula IIa :

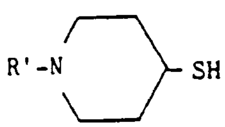

(IIa)

in which R' is as defined in claim 1, is condensed with a compound of the general formula IIIa :

Y - R (IIIa)

in which R and Y are as defined in claim 1.

**4.** A preparation process according to claim 3, characterised in that the condensation of compounds IIa and IIIa is carried out in a polar solvent at a temperature of from 80 to 110°C in the presence of an alkaline agent.

**5.** A preparation process according to claim 3, characterised in that the condensation is carried out starting from a compound IIa which has previously been sodified.

**Patentansprüche**

**Patentansprüche für folgende Vertragsstaaten : AT, BE, CH, DE, DK, FR, GB, IT, LI, LU, NL, SE**

1. Piperidin-Derivate der allgemeinen Formel (I)

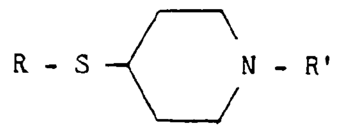

(I)

in der
- einer der Substituenten R oder R' eine Gruppe der Formel

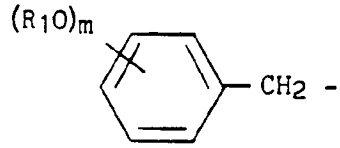

worin
- m eine ganze Zahl mit einem Wert von 1 bis 3 und
- $R_1$ eine Alkylgruppe mit 1 bis 5 Kohlenstoffatomen darstellen,

und
- der andere Substituent R oder R'
entweder eine Gruppe der Formel

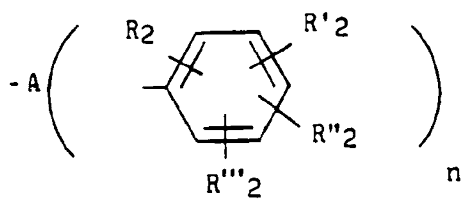

in der
- A eine Kohlenwasserstoffkette mit 1 bis 5 Kohlenstoffatomen, die gegebenenfalls eine Doppelbindung oder ein Sauerstoff- oder Schwefelatom enthält und gegebenenfalls durch eine Hydroxylgruppe oder eine oder mehrere Methylgruppen substituiert ist;
- n 1 oder 2; und
- $R_2$, $R'_2$, $R''_2$ und $R'''_2$, die gleichartig oder verschieden sein können, jeweils ein Wasserstoffatom, ein Halogenatom, wie ein Chloratom oder ein Fluoratom, eine geradkettige oder verzweigte Alkyl- oder Alkoxygruppe mit jeweils 1 bis 5 Kohlenstoffatomen, oder eine Hydroxylgruppe darstellen;

oder eine Gruppe der Formel:

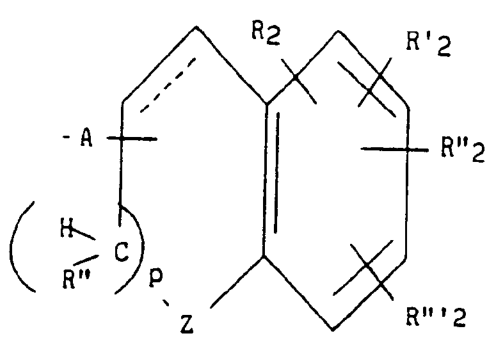

in der
- Z ein Sauerstoffatom oder ein Schwefelatom;
- R" ein Wasserstoffatom oder eine Methylgruppe;
- p 0 oder 1 darstellen;

und
- A, $R_2$, $R'_2$, $R''_2$ und $R'''_2$ die oben angegebenen Bedeutungen besitzen, bedeuten.

2. Die physiologisch verträglichen Salze der Verbindungen nach Anspruch 1 mit geeigneten Säuren.

3. 4-(2,3,4-Trimethoxy-benzyl)-thio-1-cinnamyl-piperidin.

4. 4-(2,3,4-Trimethoxy-benzyl)-thio-1-(3,5-di-tert.-butyl-4-hydroxy-benzyl)-piperidin und dessen Fumarat.

5. 4-(2,3,4-Trimethoxy-benzyl)-thio-1-[3-(2,3,5-trimethoxy-4-hydroxyphenoxy)-propyl]-piperidin und dessen Fumarat.

6. Verfahren zur Herstellung der Derivate nach Anspruch 1, **dadurch gekennzeichnet**, daß man
- ein Derivat der allgemeinen Formel II:

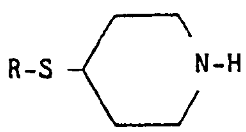

(II)

in der R die in Anspruch 1 angegebenen Bedeutungen besitzt,
- mit einem Derivat der allgemeinen Formel III:

Y - R' (III)

in der R' die in Anspruch 1 angegebenen Bedeutungen besitzt und Y ein Chloratom oder ein Bromatom oder eine Tosyloxygruppe darstellt, kondensiert.

7. Verfahren nach Anspruch 6, **dadurch gekennzeichnet,** daß man die Kondensation der Derivate II und III in einem polaren Lösungsmittel in Gegenwart eines Akzeptors für die im Verlaufe der Reaktion gebildete Wasserstoffsäure bei einer Temperatur zwischen 80 und 130°C durchführt.

8. Verfahren zur Herstellung der Derivate nach Anspruch 1, **dadurch gekennzeichnet,** daß man ein Derivat der allgemeinen Formel IIa:

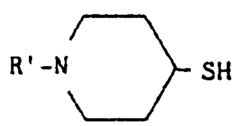

(IIa)

in der R' die in Anspruch 1 angegebenen Bedeutungen besitzt, mit einem Derivat der allgemeinen Formel IIIa:

Y-R (IIIa)

in der R die in Anspruch 1 angegebenen Bedeutungen besitzt und Y die in Anspruch 6 angegebenen Bedeutungen besitzt, kondensiert.

9. Verfahren nach Anspruch 8. **dadurch gekennzeichnet,** daß man die Kondensation der Derivate IIa und IIIa in einem polaren Lösungsmittel bei einer Temperatur zwischen 80 und 110°C in Gegenwart eines alkalischen Mittels durchführt.

10. Verfahren nach Anspruch 8, **dadurch gekennzeichnet,** daß man die Kondensation ausgehend von einem zuvor in das Natrlumderivat umgewandelten Derivat IIa bewirkt.

11. Pharmazeutische Zubereitungen enthaltend als Wirkstoff ein Derivat nach einem der Ansprüche 1 bis 5 zusammen mit geeigneten pharmazeutischen Trägermaterialien.

12. Pharmazeutische Zubereitungen nach Anspruch 11, in einer Form, die insbesondere zur Behandlung von pathologischen Zuständen geeignet ist, die mit Gewebeischämie und peripheren Gefäßerkrankungen verknüpft sind sowie zur Behandlung von Erkrankungen, bei denen eine Lipidperoxidation eine initiierende und/oder erschwerende Rolle spielt.

**Patentansprüche für folgende Vertragsstaaten : ES, GR**

1. Verfahren zur Herstellung von Piperidin-Derivaten der allgemeinen Formel I:

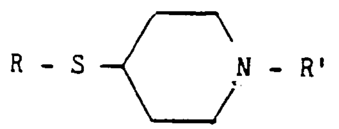

(I)

in der
- einer der Substituenten R oder R' eine Gruppe der Formel

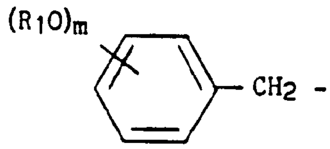

worin
- m eine ganze Zahl mit einem Wert von 1 bis 3 und
- $R_1$ eine Alkylgruppe mit 1 bis 5 Kohlenstoffatomen darstellen,

und
- der andere Substituent R oder R'

entweder eine Gruppe der Formel

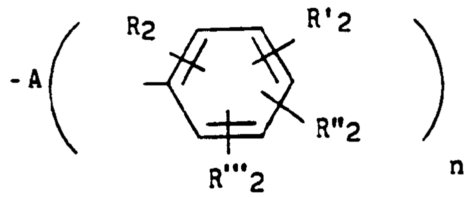

in der
- A eine Kohlenwasserstoffkette mit 1 bis 5 Kohlenstoffatomen, die gegebenenfalls eine Doppelbindung oder ein Sauerstoff- oder Schwefelatom enthält und gegebenenfalls durch eine Hydroxylgruppe oder eine oder mehrere Methylgruppen substituiert ist;
- n 1 oder 2; und
- $R_2$, $R'_2$, $R''_2$ und $R'''_2$, die gleichartig oderverschieden sein können, jeweils ein Wasserstoffatom, ein Halogenatom, wie ein Chloratom oder ein Fluoratom, eine geradkettige oder verzweigte Alkyl- oder Alkoxygruppe mit jeweils 1 bis 5 Kohlenstoffatomen, oder eine Hydroxylgruppe darstellen;

oder eine Gruppe der Formel:

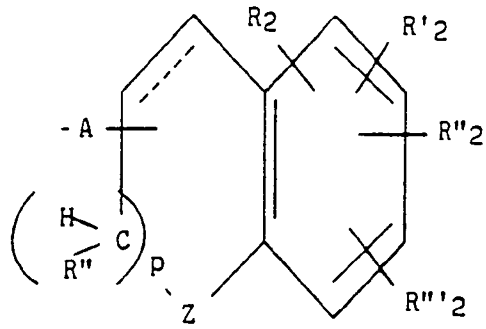

in der

- Z ein Sauerstoffatom oder ein Schwefelatom;
- R" ein Wasserstoffatom oder eine Methylgruppe;
- p 0 oder 1 darstellen;

und

- A, $R_2$, $R'_2$, $R''_2$ und $R'''_2$ die oben angegebenen Bedeutungen besitzen, bedeuten, und von deren physiologisch verträglichen Salzen mit geeigneten Säuren,

**dadurch gekennzeichnet,** daß man

- ein Derivat der allgemeinen Formel II:

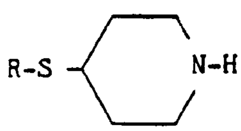

(II)

in der R die in Anspruch 1 angegebenen Bedeutungen besitzt,

- mit einem Derivat der allgemeinen Formel III:

Y-R' (III)

in der R' die in Anspruch 1 angegebenen Bedeutungen besitzt und Y ein Chloratom oder ein Bromatom oder eine Tosyloxygruppe darstellt, kondensiert.

**2.** Verfahren nach Anspruch 1, **dadurch gekennzeichnet**, daß man die Kondensation der Derivate II und III in einem polaren Lösungsmittel in Gegenwart eines Akzeptors für die im Verlaufe der Reaktion gebildete Wasserstoffsäure bei einer Temperatur zwischen 80 und 130°C durchführt.

**3.** Verfahren zur Herstellung der Derivate der allgemeinen Formel I, wie sie in Anspruch 1 definiert sind, **dadurch gekennzeichnet,** daß man ein Derivat der allgemeinen Formel IIa:

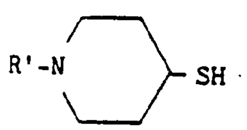

(IIa)

in der R' die in Anspruch 1 angegebenen Bedeutungen besitzt. mit einem Derivat der allgemeinen Formel IIIa:

Y-R (IIIa)

in der R und Y die in Anspruch 1 angegebenen Bedeutungen besitzen, kondensiert.

**4.** Verfahren nach Anspruch 3, **dadurch gekennzeichnet,** daß man die Kondensation der Derivate IIa und IIIa in einem polaren Losungsmittel bei einer Temperatur zwischen 80 und 110°C in Gegenwart eines alkalischen Mittels durchführt.

**5.** Verfahren nach Anspruch 3, **dadurch gekennzeichnet,** daß man die Kondensation ausgehend von einem zuvor in das Natriumderivat umgewandelten Derivat IIa bewirkt.